# EUROPEAN PATENT APPLICATION

(11) **EP 1 199 081 A1**
(43) Date of publication of application: **24.04.2002**
(21) Application number: 00203668.9
(22) Date of filing: 20.10.2000
(51) Int. Cl.: A61K 39/395, G01N 33/68, C12Q 1/68, A61P 7/02

(54) **Use of inhibition of a growth arrest-specific gene (gas6) function or of a gas6 receptor for preventing and treating a thromboembolic disease**

(71) Applicant: Vlaams Interuniversitair Instituut voor Biotechnologie, 3000 Leuven (BE); K.U. Leuven Research & Development, 3000 Leuven (BE)
(72) Inventor: Collen, Désiré, London SW5 0HN (GB); Angelillo-Scherrer, Anne, 1163 Etoy (CH); Carmeliet, Peter, 3052 Oud-Heverlee (BE)
(74) Representative: Bird, Ariane

(57) **Abstract**

Inhibition of a growth arrest-specific gene (Gas6) function or of a Gas6 receptor is used for the prevention or treatment of a thromboembolic disease or a thrombotic pathologic condition in a mammal. The invention further provides a pharmaceutical composition comprising an inhibitor of a growth arrest-specific gene (Gas6) function or of a Gas6 receptor as an active ingredient in admixture with at least a pharmaceutically acceptable carrier.

## Description

The present invention relates to a new method for the prevention and treatment of a thromboembolic disease such as arterial or venous thrombosis, based on the inhibition of, e.g. based on the administration of an inhibitor of, a growth arrest-specific gene 6 (Gas6) function or of a Gas6 receptor.

### BACKGROUND OF THE INVENTION

The major factors involved in the patho-physiology of thrombosis are abnormalities of the vessel wall, alterations of blood flow, and changes in the composition of the blood. Arterial and venous thrombosis and their complications, which include focal ischemic cerebral infarction (ischemic stroke), acute myocardial infarction and venous thromboembolism among others, represent the major cause of morbidity and death in the developed countries of the world.
Platelets play a central role in arterial thrombosis. They adhere to exposed subendothelial matrix proteins and become activated. They change their shape and then aggregate. Tissue factor (TF) is thought to be the primary initiator of *in vivo* blood coagulation. In the absence of TF expression, endothelial cells actively maintain thromboresistance. Vascular wall damage exposes TF which binds activated factor VII (factor VIIa). The factor Vlla-TF complex then triggers thrombin generation by activating factors IX and X. In addition to activating platelets, thrombin converts fibrinogen to fibrin, amplifies its own generation by activating factors V and VIII, and then activates factor XIII which finally stabilizes the fibrin clot, according to Bates et al. in *Cardiovasc. Res.* (1999) 41:418-432 and Davie E.W. in *Thromb. Haemost.* (1995) 74:1-6. Prevention and treatment of thrombosis are therefore based on the administration of either antiplatelet drugs or anticoagulants, or of a combination of both.

One of the inherited risk factors for thrombosis is protein S deficiency. Protein S, a vitamin K-dependent plasma protein, serves as a cofactor for the anticoagulant activity of an other vitamin K-dependent protein, activated protein C (APC). The protein C anticoagulant system provides important control of the blood coagulation cascade by degrading coagulation factors Va and VIIIa according to B. Dahlback in *Thromb.Haemost.* (1991) 66:49-61. Resistance to APC is the most common form of inherited thrombosis disease according to B.Dahlback in *Blood* (1995) 85:607-614.

In order to investigate the mechanism controlling growth arrest in mammalian cells, a set of six growth arrest-specific (hereinafter "Gas") genes have been cloned and sequenced. Gas6 was originally identified as a gene whose expression in mouse fibroblasts increased during serum starvation and was described in detail, together with its human homolog, by Manfioletti et al. in *Mol. Cell Biol.* (1993) 13(8):4976-4985 and U.S.Patent No. 5,538,861.

The protein encoded by Gas6 is a vitamin K-dependent protein related to protein S (i.e. human Gas6 cDNAs encode a protein having 44% amino acid sequence identity to human protein S) which is suspected to play a role in a number of biological processes, namely the regulation of a protease cascade relevant in cell growth regulation, according to Matsubara et al. in *Dev.Biol.* (1996) 180:499-510. Both molecules comprise a gamma-carboxyglutamic acid rich region (i.e. the A domain), four epidermal growth factor (EGF)-like repeats (forming the C domain) and a carboxyterminal tandem globular (G) region with homology to the steroid hormone binding globulin (SHBG) protein (i.e. the D domain). However, in contrast to protein S, Gas6 lacks a loop which is crucial for the anticoagulant activity of protein S, according to Manfioletti et al. (cited *supra).*

The Axl receptor, disclosed by O'Bryan et al. in *Mol. Cell Biol.* (1991) 11: 5016-5031, was identified due to its ability to render mouse fibroblast cells tumorigenic. Axl expression appears to have profound effects on the growth state of cells. U.S.Patent No. 5,538,861 discloses that Gas6 is a ligand for the Axl receptor. The cDNA sequence of the receptor tyrosine kinase Rse, that is preferentially expressed in the adult brain, was described by Mark et al. in *J.Biol. Chem.* (1994) 269:10720. cDNA sequences encoding proteins identical to human and human Rse have been termed Sky and Tyro3 respectively and disclosed by Ohashi et al. in *Oncogene* (1994) 9:699 and by Lai et al. in *Oncogene* (1994) 9:2567 respectively. Rse is structurally related to Axl (also known as Ufo or Ark) and shares 43% overall amino acid sequence identity with this tyrosine kinase receptor. Rse and Axl, together with c-Mer (also known as Eyk or Nyk) disclosed by Graham et al. in *Cell Growth Differ.* (1994) 5:647, define a class of tyrosine kinase receptors whose extracellular domains resemble neural cell recognition and adhesion molecules. Like Rse, Axl is also expressed in the nervous system, but is more widely expressed than Rse in peripheral tissues. Gas6 binds members of the above-mentioned class of tyrosine kinase receptors according to Nagata et al. in *J. Biol. Chem.* (1996) 271(47):30022-30027 and Crosier et al. in *Pathology* (1997) 29(2):131-135.

The extracellular domains of these receptors comprise two immunoglobulin (Ig)-like repeats followed by two fibronectin type III repeats, found in cell adhesion molecules. The Axl receptor is capable of homophilic binding as well as binding to Gas6. However, Axl is not only expressed as a transmembrane protein, but is also cleaved in the extracellular domain to generate a soluble Axl form, which has been detected in conditioned media of Axl expressing cells, serum, plasma, brain, liver, spleen and tumor cells. Soluble Axl could act as a competitive inhibitor for Gas6 by sequestering free Gas6 or could bind to Axl transmembrane receptor. Binding of soluble Axl to Axl transmembrane receptor might give a signal distinct from Gas6 or inactivate Axl transmembrane receptor on the cell surface according to Costa et al. in *J. Cell Physiol.* (1996) 168(3):737-744 and Varnum et al. in *Nature* (1995) 373:623-626.

Gas6 and Axl are expressed by vascular endothelial cells according to Varnum et al. (cited *supra).* Gas6 has been reported to inhibit homophilic Axl-mediated aggregation of myeloid cells according to Avanzi et al. in *Blood* (1998) 91(7):2334-2340, but cell-bound Gas6 may mediate aggregation of myeloid cells via interaction with Axl receptor on adjacent cells according to McCloskey et al. in *J. Biol. Chem.* (1997) 272(37):23285-23291. Gas6 does not affect adhesion of granulocytes to resting endothelial cells, while it inhibits granulocyte adhesion to TNF-α activated endothelial cells at high concentrations according to Avanzi et al. (cited *supra).* Gas6 is mitogenic for fibroblasts according to Goruppi et al. in *Oncogene* (1996) 12(3):471-480 and for Schwann cells according to Li et al. in *J. Neurosci.* (1996) 16(6):2012-9 and U.S.Patent No. 5,714,385, but not for myeloid cells according to Avanzi et al. in *Exp. Hematol.* (1997) 25(12):1219-1226 or endothelial cells. Gas6, induced in injured vascular smooth muscle cells, induces Axl-mediated chemotaxis of smooth muscle cells and, although not mitogenic by itself, enhances the mitogenic activity of thrombin according to Fridell et al. in *J. Biol. Chem.* (1998) 273(12):7123-7126. Gas6 also acts as a survival factor for serum-starved fibroblasts and GnRH neuronal cells, presumably via activation of P13-kinase and Akt kinase according to Goruppi et al. in *Mol. Cell Biol.* (1997) 17(8):4442-4453. Axl signaling protects against apoptosis as Axl deficient fibroblasts cannot be rescued by Gas6 after serum-withdrawal according to Bellosta et al. in *Oncogene* (1997) 15(20):2387-2397.

### SUMMARY OF THE INVENTION

In a first aspect, the present invention relates to the use of inhibition (for instance by means of an inhibitor or antagonist) of a growth arrest-specific gene (Gas6) function or of a Gas6 receptor as a medicine or for the manufacture of a medicine, especially a medicine for the prevention or treatment of a thromboembolic disease or a thrombotic pathologic condition in a mammal, preferably in a human. Within the framework of this invention, the growth arrest-specific gene (Gas6) receptor to be inhibited preferably is a tyrosine kinase receptor such as the Axl receptor, the Rse receptor or the c-Mer receptor. Inhibition according to this invention also includes inhibition of the native protein or polypeptide encoded by Gas6 or of a modified form thereof, for instance a form including a modified gamma-carboxyglutamic acid rich region (i.e. the A domain) - such as disclosed in U.S.Patent No. 6,017,882 - that enhances membrane binding affinity of the protein relative to the corresponding native protein.

Examples of thromboembolic diseases or thrombotic pathologic conditions within the scope of this invention namely include:
- ischemic diseases such as ischemic stroke or ischemic cerebral infarction, acute myocardial infarction, chronic ischemic heart disease.
- an ischemic disease of an organ other than myocardium or a region of the brain, for instance a peripheral limb.
- venous thromboembolism.
- arterial or venous thrombosis.
- pulmonary embolism.
- restenosis following coronary artery bypass surgery or following percutaneous transluminal angioplasty of coronary artery.
- disseminated intra-vascular coagulation.

As far as prevention is concerned, non-limiting examples of thrombotic pathologic conditions within the scope of this invention namely include, in addition to the above:
- the relapse of coronary thrombosis after acute myocardial infarction.
- coronary thrombosis in patients with unstable angina pectoris.
- cerebral ischemic infarction (ischemic stroke) in patients with atrial fibrillation.
- arterial thrombosis after vascular surgery.
- the occlusion of arterio-venous shunt in dialysis patients.

In a second aspect, the present invention relates to a pharmaceutical composition comprising an inhibitor or antagonist of a growth arrest-specific gene (Gas6) function or of a Gas6 receptor as an active ingredient in admixture with at least a pharmaceutically acceptable carrier, the said pharmaceutical composition being preferably intended for the prevention or treatment of a thromboembolic disease or a thrombotic pathologic condition, such as above defined. The said pharmaceutical composition may further optionally comprise a thrombolytic agent.

In another aspect, the present invention relates to the use of inhibition, for instance by means of an inhibitor or antagonist, of a growth arrest-specific gene (Gas6) function or of a Gas6 receptor during extracorporeal blood circulation and hemodialysis, i.e. in a method for treating blood from a mammal, in order to prevent platelet activation leading to thrombus formation in the extracorporeal system and - because of excessive platelet - bleeding in the patient. In yet another aspect, the present invention relates to the use of inhibition, for instance by means of an inhibitor or antagonist, of a growth arrest-specific gene (Gas6) function or of a Gas6 receptor as a diagnostic tool or agent, for instance in order to identify, via protein or mRNA or DNA characterization, individuals having a predisposition to acquire a a thromboembolic disease or a thrombotic pathologic condition, such as above defined.

Finally, the present invention provides a method of prevention or treatment of a thromboembolic disease or a thrombotic pathologic condition (such as above defined) in a mammal, preferably a human, comprising administering to a patient in need of such prevention or treatment a therapeutically effective amount, i.e. preferably an amount able to protect the patient against thromboembolism without causing bleeding side effects, of an inhibitor of a growth arrest-specific gene (Gas6) function or of a Gas6 receptor.

### BRIEF DESCRIPTION OF THE DRAWINGS

Figure 1 shows the effect of Gas6 deficiency or of anti-Gas6 antibodies on platelet aggregation.
Figure 2 shows the aggregation of wild-type (+/+) and gas6 deficient (-/-) platelets to thrombin, phorbol-12-myristyl-13-acetate (PMA) and the Ca⁺⁺ ionophore A23187.

### DETAILED DESCRIPTION OF THE INVENTION

We have now found that Gas6 deficient mice are resistant to thrombosis, induced by venous stasis, arterial denudation or collagen/epinephrine injection - models known to depend to a variable degree on blood coagulation and platelet aggregation according to Di Minnio et al. in *J. Pharmacol. Exp. Ther.* (1983) 225:57-60, Herbert et al. in *Blood* (1992) 80:2281-6 and Matsuno et al. in *Br. J. Pharmacol.* (1992) 106:533-8. Their resistance to thrombosis was not due to differences in coagulation or thrombolysis, nor to abnormalities in the number or morphology of platelets. Instead, loss of Gas6 caused platelet dysfunction. Indeed, Gas6, while ineffective by itself, significantly enhanced the formation of stable platelet aggregates by several platelet agonists such as adenosine 5'-diphosphate (ADP), collagen and the thromboxane A₂ (TX A₂) analogue U46619. In the absence of Gas6, low concentrations of these agonists could only induce reorganization of actin filaments that cause the shape changes preceding initial formation of platelet aggregates. Signaling by the ADP-, collagen-, TXA2- or thrombin-receptors was not completely blocked in Gas6 deficient platelets, since shape change did occur in response to low concentrations of the platelet agonists and irreversible platelet aggregation proceeded in response to high concentrations of these agonists. Only thrombin induced aggregation of Gas6 deficient platelets at low concentrations, but adenosine 5'-triphosphate (ATP) release induced by thrombin was lower in Gas6 deficient platelets than in wild type platelets. The downstream pathways mediating granule secretion and platelet aggregation according to Rao et al. in *Arterioscl. Thromb. Vasc. Biol.* (2000) 20:285-289 were operational in Gas6 deficient platelets, since PMA or the Ca⁺⁺ ionophore A23187 induced normal secretion and aggregation. Thus, in Gas6 deficient platelets both secretion of granules and aggregation of platelets occurred less efficiently.
An autocrine role for Gas6 in platelets is suggested by the finding that Gas6 is present in -granules and, following platelet activation, becomes secreted and bound to the platelet surface, most likely via Gas6 receptors. Since Gas6 deficient platelets have normal expression of the Gas6 receptors Axl and Sky, the platelet defects were not related to downregulation of these receptors. Collectively, our data are consistent with a model in which Gas6 is released from the α-granules upon initial stimulation of platelets by several agonists. Subsequently, Gas6 amplifies, via signaling through one or more of its receptors, the intracellular signals generated via the ADP-, collagen-, TXA₂- and thrombin-receptors. Gas6 exerts this amplification signal at the level or downstream of the platelet agonist receptors, but likely upstream of protein kinase C activation or Ca⁺⁺ mobilization.

The phenotype of Gas6 deficient mice resembles several features of patients with platelet signal transduction defects. Like Gas6 deficient mice, these patients have impaired secretion of dense granules in response to weak agonists or to low concentrations of potent agonists. The number of platelet granules, TXA₂ production and initial aggregation are normal according to Rao et al. (cited *supra).* The findings of the present invention therefore suggest Gas6 defects as a mechanism of these primary signal transduction defects.

Gas6 appears to be redundant for baseline hemostasis but constitutes an important "amplification" system in pathological conditions. Because Gas6 only amplifies the response of other platelet agonists, but does not evoke a response itself, inhibition of Gas6 constitutes an attractive treatment to prevent thrombosis without causing bleeding side-effects. Indeed anti-Gas6 antibodies protected wild type mice against fatal thromboembolism to the same degree as genetic inactivation of Gas6, while not causing spontaneous bleeding, implying a therapeutic efficiency of Gas6 inhibitors in the treatment of thrombotic disorders. By modulating - but not completely blocking - signaling of the principal platelet agonists, Gas6 antagonists are believed to be safer than the currently available antiplatelet drugs. Anti-Gas6 antibodies may be obtained by screening test inhibitory compounds from large libraries of synthetic or natural compounds. Synthetic compound libraries are commercially available from e.g. Maybridge Chemical Co. (Trevillet, Cornwall, UK), Comgenex (Princeton, NJ), Brandon Associates (Merrimack, NH), Microsource (New Milford, CT) and Aldrich Chemical Company, Inc. (Milwaukee, WI). Alternatively, libraries of natural compounds in the form of bacterial, fungal, plant and animal extracts are available from e.g. New Chemical Entities, Pan Laboratories, Bothell, MycoSearch and Chiron Inc. Additionally, synthetic and natural inhibitory compounds obtained from such libraries may be readily modified through conventional chemical, physical and biochemical means. An illustrative example of an anti-Gas6 antibody suitable for the performance of the present invention is constituted by the antibody referenced as 620SC_1935 in the catalogue from Santa Cruz Biotechnology, Santa Cruz, California, directed against the carboxy-terminal part of Gas6.

These data show that inhibitors or antagonists of the Gas6 function or of a Gas6 receptor can be used as medicines, especially for the prevention and/or treatment of a thromboembolic disease or a thrombotic pathologic condition, such as arterial and/or venous thrombosis, in a mammal. They appear to constitute a new class of promising antithrombotic drugs with reduced bleeding tendency. In view of bioavailability, said inhibitors or antagonists are preferably used as active ingredients in pharmaceutical compositions further comprising a pharmaceutically acceptable carrier. Suitable pharmaceutical carriers for use in the pharmaceutical compositions of the invention are described for instance in Remington's Pharmaceutical Sciences 16^{th} ed. (1980) and their formulation is well known to those skilled in the art. They include any and all conventional solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition comprising the active ingredient may require protective coatings as are well known in the art. The pharmaceutical form suitable for injectionable use include sterile aqueous solutions or dispersions and sterile powders for the extemporaneous preparation thereof. Typical carriers therefor include biocompatible aqueous buffers, ethanol, glycerol, propylene glycol, polyethylene glycol and mixtures thereof. The pharmaceutical compositions of the invention may be suitably formulated, using formulating methods well known to those skilled in the art, for oral, intranasal, subcutaneous, intramusvular, intradermal, intravenous, intraarterial or parenteral administration or for catheterization.

The pharmaceutical compositions of the invention may further comprise a therapeutically effective amount of at least one known thrombolytic agent. Thrombolytic agents which may namely be considered for this purpose include fibrin-specific agents such as wild-type tissue-type plasminogen activator and mutants and variants thereof, single-chain urokinase-type plasminogen activator and staphilokinase, and non-fibrin-specific agents such as two-chain urokinase-type plasminogen activator, streptokinase and anisoylated plasminogen streptokinase activator complex (anistreplase). All of them are well documented by R.Lijnen et al. in *Cardiovascular Thrombosis* (1998) pp.301-315, 2^{nd} ed., Linpicott-Raven Publishers (Philadelphia).

The method of prevention or treatment of a thromboembolic disease or a thrombotic pathologic condition in a mammal according to the invention may, in addition to administering a therapeutically effective amount of an inhibitor of a growth arrest-specific gene (Gas6) function or of a Gas6 receptor, further comprise administering to the patient a therapeutically effective amount of at least one known thrombolytic agent such as above described. The latter administration may be either simultaneous or sequential with regard to administration of the Gas6 active component.

The present invention will be demonstrated in more detail in the following examples, which are however not intended to limit the scope of the invention.

### EXAMPLE 1 - MICE DEFICIENT IN GAS6 (GAS6^{-/-} MICE) ARE RESISTANT TO STASIS-INDUCED THROMBOSIS

Animal experiments were conducted according to the guiding principles of the American Physiological Society and the International Committee on Thrombosis and Haemostasis as disclosed by A. Giles in *Thromb. Haemost.* (1987) 58:1078-1084.

Thrombus formation by stasis was induced as described by Vogel et al. in *Thromb. Res.* (1989) 54:399-410. Briefly, wild type mice (Gas6^{+/+} mice) or mice in which Gas6 expression was abolished by homologous recombination (Gas6^{-/-} mice), of either sex, with a genetic background of 50% Swiss/50% 129, weighing 20 to 30 g, were anesthetized by intra-peritoneal injection of 60 mg/kg sodium pentobarbital. The abdomen of the animal was opened surgically and, after careful dissection, the vena cava was exposed and dissected free from surrounding tissue. Two loose sutures were prepared 0.7 cm apart on the inferior vena cava and all collateral veins were ligated. Stasis was established by tightening the two sutures, first the proximal and then the distal. The abdominal cavity was closed provisionally and stasis was maintained for 20 minutes. The cavity was then reopened, the ligated segment was opened longitudinally and the thrombus formed was removed, rinsed, blotted on filter paper, dried overnight at 60°C and weighed.

The data are represented as mean ± SEM of n determinations. The significance of differences was determined by unpaired t-test. In Gas6^{+/+} mice, thrombus weight was 2.46 ± 0.24 mg (n= 10). In contrast, thrombus weight measured in Gas6^{-/-} mice was 0.38 ± 0.08 mg (n= 10) (84.6% inhibition in Gas6^{-/-} mice, p<0.001). These data indicate that the lack of Gas6 expression induced resistance to stasis-induced thrombosis.

### EXAMPLE 2 - MICE DEFICIENT IN GAS6 (GAS6^{-/-} MICE) ARE RESISTANT TO THROMBOSIS IN THE CAROTID ARTERY PHOTOCHEMICALLY INDUCED BY ROSE-BENGAL

Mice were anesthetized by intraperitoneal injection of 60 mg/kg sodium pentobarbital and then fixed on heated operating table. Atropine sulphate was injected in all animals subcutaneously (0.5 mg/kg), and endotracheal intubation was carried out. A 2F venous catheter was inserted into the right jugular vein for injection of rose bengal. The left carotid artery was exposed and mounted on an appropriate transilluminator. Thrombus formation was induced by a photochemical reaction according to the method of Umemura et al. in *Thromb. Haemost.* (1996) 76:799-806.

Briefly, the exposed artery was irradiated with green light (wavelength 540 nm) of a Xenon lamp (L4887, Hamamatsu Photonics, Hamamatsu, Japan) equipped with a heat-absorbing filter and a green filter. Irradiation was directed via a 3 mm diameter optic fiber attached to a manipulator. Rose bengal was administered via an intravenous (slow) bolus injection in a total volume of 200 I. Irradiation was started just after injection and was maintained for 4 minutes according to Kawasaki et al. in *Throm. Haemost.* (1999) 81:306-11.

In Gas6^{+/+} mice, thrombus mass was 380 ± 91 x 10³ light units (mean ± SEM, n=5). In contrast, thrombus mass measured in Gas6^{-/-} mice was 160 ± 35 x 10³ light units (mean ± SEM, n=5), (p<0.05). These data indicate that the lack of Gas6 expression induced resistance to arterial thrombosis photochemically induced by rose bengal.

### EXAMPLE 3 - MICE DEFICIENT IN GAS6 (GAS6^{-/-} MICE) ARE PROTECTED AGAINST COLLAGEN/EPINEPHRIN-INDUCED THROMBOEMBOLISM

A mixture of collagen (0.5 mg/kg, equine collagen, Kollagenreagent Horm, available from Hormon Chemie, Munich, Germany) and epinephrine (60 g/kg) was injected into the jugular vein of mice anesthetized by intraperitoneal injection of 60 mg/kg sodium pentobarbital according to Di Minnio et al. in *J. Pharmacol. Exp. Ther.* (1983) 225:57-60.

The mortality within 15 minutes induced by infusion of a collagen/epinephrin mixture was 80% in Gas6^{+/+} mice (n=10) versus 20% in GAS6^{-/-} mice (n=10) (p<0.03). These data indicate that the lack of Gas6 expression induced resistance to collagen/epinephrin thromboembolism.

### EXAMPLE 4 - PLATELET AGGREGATION IS DEFECTIVE IN GAS6 DEFICIENT MICE (GAS6^{-/-} MICE)

From mice anesthetized by intraperitoneal injection of 60 mg/kg sodium pentobarbital, whole blood was drawn from the inferior vena cava into 0.1 M citrate (1 volume anticoagulant/ 9 volumes of blood). Blood was centrifuged at 100g for 10 minutes, allowing separation of platelet-rich plasma (PRP). Platelet-poor plasma (PPP) was obtained by centrifugation of the remaining blood at 2,000g for 10 minutes. PRP and PPP were pooled from four Gas6^{-/-} or Gas6^{+/+} mice. Platelet aggregation was measured turbidimetrically in an optical Chronolog aggregometer (model 490, Coulter Electronics Ltd), using 280 µl PRP, adjusted to a concentration of 250,000 platelets/µl with PPP as a diluent. PPP also served as 100% reference for aggregation. Aggregation in response to collagen (equine collagen from Hormon Chemie), ADP or the thromboxane A2 mimetic U46619 was studied.

Aggregation in response to thrombin was performed with washed platelets. Briefly, blood was drawn from the inferior vena cava into acid-citrate-dextrose solution (ACD) (1 volume ACD / 6 volume blood) and PRP was pooled from four Gas6^{-/-} or Gas6^{+/+} mice. Apyrase was added to PRP at a final concentration of 1 u/ml. Platelets were then washed by adding 2 volumes ACD and pelleted by centrifugation at 2,000 g for 10 minutes. Platelet pellet was resuspended in Tyrode's buffer containing 1% BSA and final platelet suspension was adjusted to 200,000 platelets/µl and kept at 37°C. Platelet aggregation was measured with an optical Chronolog aggregometer as described above.

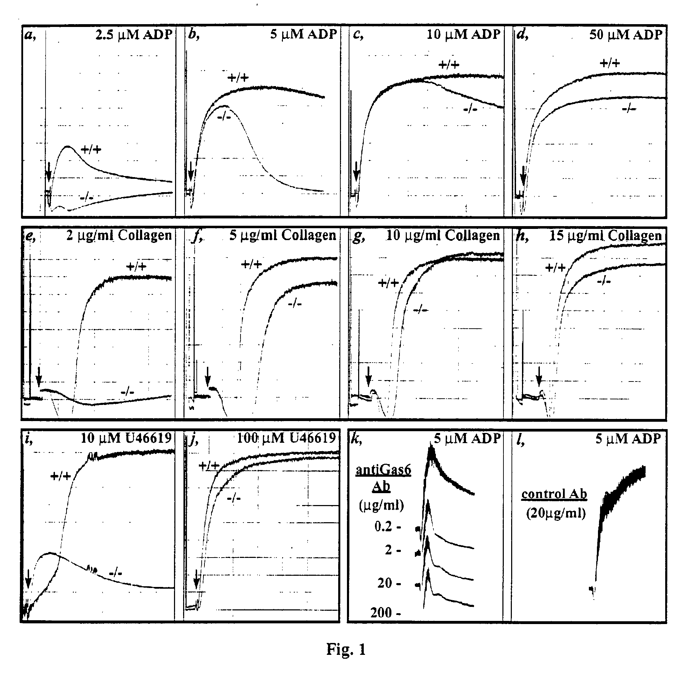

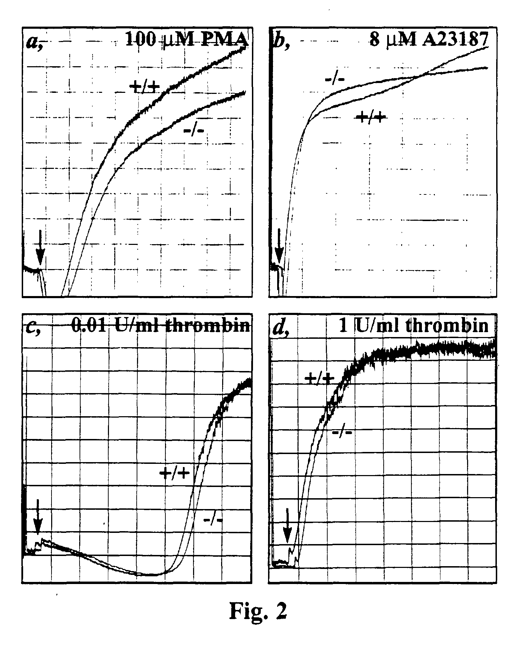

As shown in figures 1 and 2, platelet aggregation studies revealed significant functional defects in Gas6^{-/-} mice. Platelets from Gas6^{+/+} mice dose-dependently aggregated in response to ADP (fig.1*a-d*), collagen (fig.1*e-h*) or the thromboxane A₂ analogue U46619 (fig.1*i,j*). Maximal aggregation was achieved at similar concentrations as used previously by Offermans et al. in *Nature* (1997) 389:183-186. In contrast, platelets from Gas6^{-/-} mice failed to irreversibly aggregate in response to low concentrations of ADP (<10 µM), collagen (2 µg/ml) and U46619 (10 µM). At low agonist concentration, Gas6^{-/-} platelets only displayed shape change as revealed by an immediate decrease in light transmission after stimulation. However, higher concentrations of ADP (50 µM in fig. 1*d*), collagen (5-15 µg/ml in fig.1*f-h*) or U46619 (100 µM in fig.1*j*) induced irreversible aggregation of Gas6^{-/-} platelets. Both Gas6^{+/+} and Gas6^{-/-} platelets aggregated normally in response to phorbol-12-myristyl-13-acetate (PMA) or the Ca⁺⁺ ionophore A23187 (fig.*2a,b*). Thrombin stimulated platelet aggregation comparably in both genotypes at all doses tested (fig.*2c,d*). In both figures 1 and 2, squares represent 2 minutes (on X-axis) and 10% change in light transmission (on Y-axis). The arrow in each panel indicates the application of the platelet agonists (in fig.1), PMA or thrombin (in fig.2).

### EXAMPLE 5 - PLATELET SECRETION IS DECREASED IN GAS6^{-/-} MICE

Platelet aggregation and, ATP secretion were measured in an optical Chronolog Lumi-aggregometer (Coulter Electronics Ltd), using 280 µl PRP, adjusted to a concentration of 250,000 platelets/µl with PPP as a diluent. Platelet aggregation was measured as described in example 4. Platelet ATP release was monitored by adding firely luciferase and luciferin to all samples and comparing luminescence created by platelet ATP release to that generated by addition of an ATP standard (Chrono-Lume, Kordia). Aggregation and ATP release in response to collagen (equine collagen from Hormon Chemie), ADP, or the thromboxane A2 mimetic U46619 were studied. Platelet aggregation and ATP secretion in response to thrombin were performed with washed platelets in an optical Chronolog Lumi-aggregometer as described above.

Secretion of ADP and ATP from dense granules is essential for the formation of stable macroaggregates after initial formation of small, unstable platelet aggregates as described by A.J.Marcus, Disorders of Hemostasis (1997) 79-137 (eds. Ratnoff & Forbes). Secretion of dense granule stores (evaluated by measuring release of ATP) was significantly impaired in Gas6^{-/-} platelets. Compared to Gas6^{+/+} platelets, release of ATP from Gas6^{-/-} platelets was significantly decreased in response to ADP, collagen or U46619, when these agonists were used at low concentrations (only causing platelet shape changes or reversible platelet aggregation) as shown in following Table 1. ATP release from Gas6^{-/-} platelets was also reduced in response to high concentration of ADP (50 µM) or thrombin (1 U/ml). However, release of ATP was normal or only slightly reduced when Gas6^{-/-} platelets were stimulated with high concentrations of collagen (10 µg/ml) or U46619 (100 µM) (see Table 1), which cause irreversible platelet aggregation. PMA and the Ca⁺⁺ ionophore A23187 induced a normal secretion response in both genotypes (see Table 1). Thus, a close correlation was found between the defects in the aggregation (see also example 4) and ATP secretion response of Gas6^{-/-} platelets to various agonists.

**Table 1**

| Agonist | concentration | Gas6^{+/+} | Gas6^{-/-} |
|---|---|---|---|
| ADP | 20 µM | 0.8 ± 0.1 | 0.2 ± 0.1* |
| | 50 µM | 2.6 ± 0.8 | 1.3 ± 0.3* |
| Collagen | 1 µg/ml | 1.8 ± 0.3 | ND |
| | 10 µg/ml | 10 ± 1.0 | 11 ± 0.8 |
| U46619 | 10 µM | 6.5 ± 1.5 | ND |
| | 100 µM | 8.2 ± 2.0 | 7.6 ± 2.3 |
| Thrombin | 1 U/ml | 17 ± 1.6 | 11 ±1.2* |
| PMA | 100 µM | 2.3 ± 0.4 | 1.9 ± 0.6 |
| A23187 | 8 µM | 9.2 ± 3.1 | 7.3 ± 3.0 |

In Table 1, the data represent mean ± SEM of three experiments using platelet-rich plasma (for ADP, collagen, U46619, PMA or A23187 stimulation) or washed platelets (for thrombin stimulation). Each experiment was performed with a pool of 4 to 6 Gas6^{+/+} or Gas6^{-/-} mice. ND means not detectable.

### EXAMPLE 6 - ANTI-GAS6 ANTIBODIES INHIBIT PLATELET AGGREGATION

Blood from human volunteers was collected from the antecubital vein (9 volumes) and anticoagulated with 3.8% citrate (1 volume). PRP was obtained by centrifugation of citrated whole blood at 120g for 15 minutes. Washed platelets were prepared as mentioned in example 4. The effect of Gas6-specific antibodies was studied on platelet aggregation *in vitro.* Antibodies (available from Santa Cruz Biotechnology, Santa Cruz, California) directed against the carboxyterminal part of Gas6 - responsible for binding of Gas6 to its receptors according to Mark et al. in *J.Biol.Chem.* (1996) 271:9785-9 - were used. Platelet aggregation was measured turbidimetrically in an optical Chronolog aggregometer (model 490, Coulter Electronics Ltd), using 280 µl PRP, adjusted to a concentration of 250,000 platelets/µl with PPP as a diluent. PPP also served as 100% reference for aggregation. Platelets were pre-incubated with Gas6 neutralizing antibodies (at concentrations of 0.2, 2, 20 and 200 µg/ml respectively) or isotype-matched control antibodies (at a concentration of 20 µg/ml) for 15 seconds at 37°C before induction of aggregation with ADP (5 µM). Gas6 neutralizing antibodies dose-dependently blocked aggregation of washed human platelets in response to ADP (5 µM) as shown in fig.1*k-l*. None of the antibodies had any effect on platelets in the absence of ADP.

### EXAMPLE 7 - ANTI-GASµ6 ANTIBODIES PROTECT MICE AGAINST COLLAGEN/EPINEPHRIN-INDUCED THROMBOEMBOLISM

Mice where injected through the tail vein with either 100 µg goat polyclonal antibodies directed against the carboxyterminal part of human Gas6 or irrelevant isotype-matched antibodies 30 minutes before the thromboembolism challenge. Thromboembolism was then induced by injecting a mixture of collagen (0.5 mg/kg, equine collagen from Hormon Chemie) and epinephrine (60 µg/kg) into the jugular vein of mice anesthetized by intraperitoneal injection of 60 mg/kg sodium pentobarbital.

The mortality within 15 minutes induced by infusion of a collagen/epinephrine mixture was 80% in wild-type mice pre-injected with irrelevant isotype-matched antibodies (n=16) versus 25% in wild-type mice pre-injected with anti-Gas6 antibodies (n=12) (p<0.03). Thus, Gas6-neutralizing antibodies protected wild-type mice against fatal collagen/epinephrine-induced thromboembolism to the same degree as genetic loss of Gas6. Gas6 antibody-treated mice did not show any signs of bleeding. These results indicate that inhibition of Gas6 effectively blocks thrombosis.

### EXAMPLE 8 - GAS6 DEFICIENT MICE (GAS6^{-/-} MICE) HAVE NO SPONTANEOUS BLEEDING AND NORMAL BLEEDING TIME

No spontaneous bleeding tendency has been observed in Gas6^{-/-} mice. The morphology of Gas6^{-/-} platelets on blood smear was normal. Bleeding time (performed by tail transsection according to Dejana et al. in *Thromb. Haemost.* (1982) 48:108-111) was comparable in Gas6^{+/+} and Gas6^{-/-} mice (166 ± 48 µl, n=10 and 172 ± 68 µl, n=10, respectively, p>0.05). Thus, inhibitors of Gas6 function and of Gas6 receptors, in particular tyrosine kinase receptors such as the Axl, the Rse or the c-Mer receptor, appear to represent a new class of antithrombotic drugs with a favorable antithrombotic/bleeding ratio.

## Claims

1. Use of inhibition of a growth arrest-specific gene (Gas6) function or of a Gas6 receptor for the prevention or treatment of a thromboembolic disease or a thrombotic pathologic condition in a mammal.

2. Use according to claim 1, wherein inhibition is effected by means of an inhibitor or antagonist of a growth arrest-specific gene (Gas6) function or of a Gas6 receptor.

3. Use according to claim 1 or claim 2, wherein the said Gas6 receptor is a tyrosine kinase receptor.

4. Use according to any of claims 1 to 3, wherein the said Gas6 receptor is selected from the Axl receptor, the Rse receptor and the c-Mer receptor.

5. Use according to any of claims 1 to 4, wherein the said thromboembolic disease or thrombotic pathologic condition is selected from an ischemic disease, ischemic stroke or ischemic cerebral infarction, acute myocardial infarction, chronic ischemic heart disease, an ischemic disease of an organ other than myocardium or a region of the brain, venous thromboembolism, arterial or venous thrombosis, pulmonary embolism, restenosis following coronary artery bypass surgery or following percutaneous transluminal angioplasty of coronary artery.

6. Use of inhibition of a growth arrest-specific gene (Gas6) function or of a Gas6 receptor during extracorporeal blood circulation and hemodialysis.

7. Use of inhibition of a growth arrest-specific gene (Gas6) function or of a Gas6 receptor in order to identify, via protein or mRNA or DNA characterization, individuals having a predisposition to acquire a thromboembolic disease or a thrombotic pathologic condition.

8. A pharmaceutical composition comprising an inhibitor of growth arrest-specific gene (Gas6) function or of a Gas6 receptor as an active ingredient in admixture with at least a pharmaceutically acceptable carrier.

9. A pharmaceutical composition according to claim 8 for the prevention or treatment of a thromboembolic disease or a thrombotic pathologic condition.

10. Use of an inhibitor of a growth arrest-specific gene (Gas6) function or of a Gas6 receptor as a medicine.
